# EUROPEAN PATENT APPLICATION

(11) **EP 1 977 779 A2**
(43) Date of publication of application: **08.10.2008**
(21) Application number: 08101546.3
(22) Date of filing: 12.02.2008
(51) Int. Cl.: A61M 16/04

(54) **Endotracheal tube**

(30) Priority: 26.02.2007 RU 2007107188 U
(71) Applicant: Pervak, Konstantin Anatolievich, Vladivostok 690018 (RU); Pervak, Vladimir Anatolievich, St. Petersburg 191040 (RU)
(72) Inventor: Pervak, Konstantin Anatolievich, Vladivostok 690018 (RU); Pervak, Vladimir Anatolievich, St. Petersburg 191040 (RU)
(74) Representative: Ucinska, Julita

(57) **Abstract**

The present utility model relates to devices and appliances for introducing drugs into the human upper air passages. An endotracheal tube contains a flexible pipe (1), a connector (16) on the proximal end, a distal part of the flexible pipe (1) comprising: an inflatable cuff (2); a longitudinal air channel (10); a number of injection holes (7,9) opening on the exterior surface of the flexible pipe (1); a longitudinal channel (11) and radial ring channels (12,13) for supply of drugs. An indicator ring is placed on a site of location of the injection holes (7) along the diameter of the flexible pipe (1), and a color of the indicator ring differs from a color of the flexible pipe (1). This indicator ring is located so that it can be visible from the side of the laryngopharynx in working position of the endotracheal tube, being in this case at the boundary (15) of the larynx upper part (14). It is better when a total hydraulic resistance of one radial ring channel (12) together with the injection holes (7) made in it is equal to a total hydraulic resistance of another radial ring channel (13) together with injections holes (9) made in it.

## Description

The utility model relates to the field of medicine, namely, to devices and appliances for introducing drugs into the human upper air passages.

It is known an endotracheal tube containing a cuff, cuff-inflating catheter, connector to connect the endotracheal tube with a machine for artificial lung ventilation, cylinder to monitor and optimize pressure in the cuff (patent RU 2150300, published on June 10, 2000). In the endotracheal tube wall in the immediate vicinity of the cuff there are two radial ring channels and holes connecting the channels with the exterior of the endotracheal tube, and the endotracheal tube itself is equipped with a catheter to supply local anesthetic, connected by one of its ends with each of the radial ring channels, through one of which the local anesthetic is supplied to the area of contact of the cuff with the larynx mucous membrane and to underlaying parts of air passages, and through the second one - to the laryngopharynx area. When the known endotracheal tube is introduced into the upper air passages there is a need to control a depth of introducing the tube and to regulate its desirable position with respect to respiratory apparatus.

The technical problem in hand is to improve the construction of the endotracheal tube with an inflatable cuff in order to increase accuracy and uniformity of injection of drugs into the larynx at the expense of making a way for fast control over accuracy of positioning of the endotracheal tube in its working position and making conditions for uniform injection of drugs into the larynx.

We propose an endotracheal tube containing a flexible pipe, a connector on a proximal end of the flexible pipe to connect to a machine for artificial lung ventilation, a distal part of the flexible pipe, which includes: an inflatable cuff around the distal part to keep the distal part in the larynx in a desirable position; a longitudinal air channel in a wall of the flexible pipe to connect a cavity of the inflatable cuff with a means for inflating the cuff; a longitudinal channel for supply of drugs in the wall of the flexible pipe; a radial ring channel or channels for supply of drugs in the wall of the flexible pipe connected with the longitudinal channel for supply of drugs, and a number of injection holes made in the exterior wall of the radial ring channel or channels. New features are that the distal part of the flexible pipe further includes an indicator ring placed on the exterior of the distal part of the flexible pipe on a site of location of the injection holes or in the immediate vicinity to the site along a diameter of the flexible pipe; where a color of the indicator ring differs from a color of the exterior, at least, of the distal part of the flexible pipe; the indicator ring is located so that it can be visible from the side of the laryngopharynx in working position of the endotracheal tube, being in this case at the boundary of the larynx upper part.

One end of the longitudinal channel for supply of drugs can be connected with a means for supply of drugs, and the second end of the longitudinal channel is connected with the radial ring channel or channels.

It is preferable, when the longitudinal air channel and the longitudinal channel for supply of drugs are located diametrically opposite one to another within the wall of the flexible pipe.

Depending on therapeutic purpose, there can be two or more radial ring channels, and a total hydraulic resistance of one radial ring channel together with the injection holes made in it should be equal to a total hydraulic resistance of another radial ring channel together with the injections holes made in it, or every of other radial ring channels together with the injection holes made in it.

The exterior wall of the radial ring channel for supply of drugs can be made as a protruding ring collar of the flexible pipe. In this case it is preferable, when the indicator ring is made on the ring collar.

The injection holes can be directed perpendicularly to the axes of the distal part of the flexible pipe and/or at an acute angle to the axes of the distal part of the flexible pipe towards the inflatable cuff.

There can be three or more injection holes made in the exterior wall of the radial ring channel, and it is desirable when they locate with an equal step on the diameter of the radial ring channel.

The present utility model is explained by figures including:
Fig.1 - general view of the endotracheal tube in accordance with the preferable embodiment of invention; and
Fig.2 - local view A from Fig.1 in section on an enlarged scale, showing the distal part and its placement in working position.

The present utility model is explained by the example of preferable embodiment.

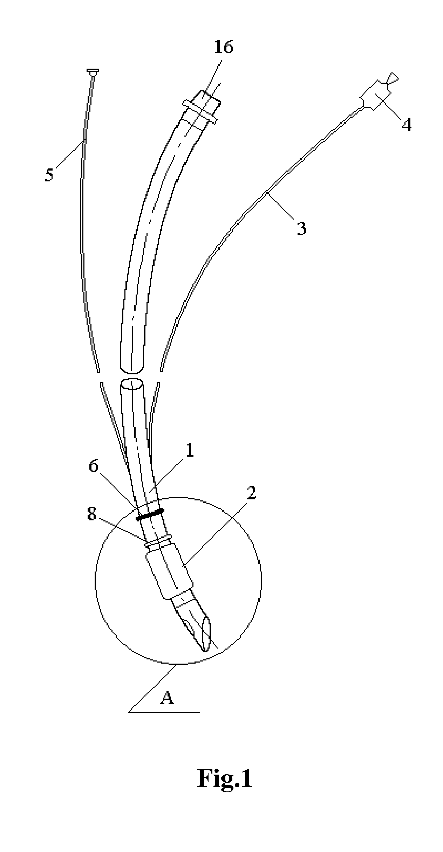

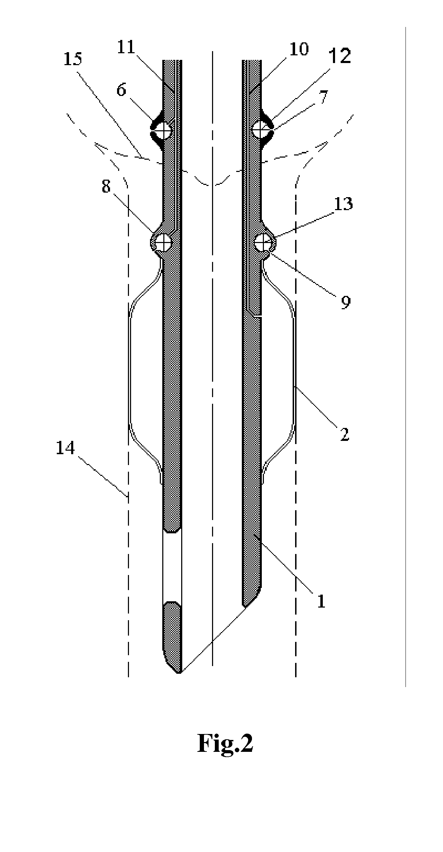

The endotracheal tube (see Fig.1 and Fig.2) incorporates a flexible pipe 1 having the inner diameter 8 mm with connector 16 on its proximal end for connection with the machine for artificial lung ventilation (not shown), including on its distal part: an inflatable cuff 2 having diameter 28 mm when inflated (i.e. to the usual diameter of trachea 14); a longitudinal air channel 10 having an inner diameter 0,5-1,5 mm, connected through a catheter 3 with a pneumatic cylinder 4 to inflate the inflatable cuff 2; a longitudinal channel for supply of drugs 11 having an inner diameter 1-2 mm, connected through a catheter 5 with a means for supply of local anesthetic in liquid and/or aerosol form (not shown); a first radial ring channel 12 having an inner diameter 0,5-1,5 mm, connected with the channel 11 and having an exterior protruding wall as a ring collar 6, which exterior acts as indicator ring, which is mated smoothly with the exterior of the distal part of the flexible pipe 1 and equipped with three or more through injection holes 7 having diameters 0,1-0,4 mm, located with an equal step to the diameter of collar 6; a second radial ring channel 13 having an inner diameter 0,5-1,5 mm, connected with the channel 11 and having an exterior protruding wall as a second ring collar 8 mated smoothly with the exterior of the distal part of flexible pipe 1 and equipped with three or more through injection holes 9 having diameters 1-0,4 mm, located with an equal step to the diameter of the collar 8.

It is better when internal diameters of the ring channels 12 and 13 are equal to each other and equipped with the same quantity of the injection holes 7 and 9 of the same diameter, in so doing, the total hydraulic resistance of the ring channel 12 together with the injection holes 7 is equal to the total hydraulic resistance of the ring channel 13 together with the injection holes 9, this enables to smoothly inject a drug from all injection holes 7 and 9 simultaneously. It is better to direct the injection holes 7 perpendicularly to the axes of the distal part of the flexible pipe 1, and the injection holes 9 would be directed at an acute angle to the axes of the distal part of flexible pipe 1 towards the inflatable cuff 2 at an angle of 30-60°.

It is better when all elements of the endotracheal tube are made of unpainted polymeric materials, for example, polyvinylchloride, and the inflatable cuff 2 is made of silicone, at that, the collar 6 acting as the indicator ring is painted from the outside or across it width to a bright red or blue color. There may be a variant when the collar 6 is painted or incorporates a pigment visible in MRT, CT, roentgenoscopy and other ways of observation for the location of this collar 6. It is desirable for adult patients to locate the collar 6 from the top edge of the inflatable cuff 2 at a distance of 30-45 mm, and to locate the collar 8 closer to the edge of the inflatable cuff 2 at a distance of 0-2 mm from it. In addition, longitudinal movement of the inflatable cuff 2 along the length of the distal part of the flexible pipe 1 may be provided. A number of the injection holes 7 may vary from 2 up to 15.

For use, the distal part of the flexible pipe 1 is inserted into the larynx in such way as to arrange the collar 6 in the laryngopharynx in a required position, residing in this case at the boundary of the upper part of the larynx 15, which is supervised visually. Such position of the collar 6 meets the proper position of the endotracheal tube, wherein the injection holes 7 are directed to the laryngopharynx zone to sub-ligamentous space, and the collar 8 is directly in the upper part of the larynx 14. Local anesthetic as solution or aerosol should be injected immediately through the channel 11 by means of the catheter 5, in so doing, the local anesthetic over the channels 11 and 12 through the injection holes 7 is directly supplied to the laryngopharynx zone to sub-ligamentous space, and is simultaneously supplied over the channels 11 and 13 through the injection holes 9 to the zone of contact of the inflatable cuff 2 with the mucous surface of the larynx 14, as well as to underlaying parts of air passages. Then, the inflatable cuff 2 is inflated through the catheter 3 and the channel 10, resulting in firmly positioning the distal part of the flexible pipe 1 in the larynx 14. Should the need arise to inject local anesthetic repeatedly to the zone of contact of the inflatable cuff 2 with the mucous surface of the larynx 14 and/or to underlaying parts of air passages, air pressure in the inflatable cuff 2 should be pre-relieved, and a proper position of the endotracheal tube should be monitored by means of visual control over the above described required position of the collar 6.

Thus, when intubating the trachea, exact, smooth and effective anesthesia of the mucous surface of the larynx and laryngopharynx is taking place, preventing reflex hemodynamic reactions. As a consequence, doses of general anesthetics and dozes of muscle relaxants are reduced, assisting in the savings in drugs, decrease in intoxication of the patient organism and more successful postoperative period.

The given example is used only to illustrate a possibility of embodiment of the utility model and in no way restricts a scope of legal protection presented in the claims; at that, a specialist in this area of engineering is easily capable of implementing other ways of embodiment of the utility model.

## Claims

1. An endotracheal tube containing a flexible pipe, a connector on a proximal end of the flexible pipe to connect to a machine for artificial lung ventilation, a distal part of the flexible pipe, which includes: an inflatable cuff around the distal part to keep the distal part in the larynx in a desirable position; a longitudinal air channel in a wall of the flexible pipe to connect a cavity of the inflatable cuff with a means for inflating the cuff; a longitudinal channel for supply of drugs in the wall of the flexible pipe; a radial ring channel or channels for supply of drugs in the wall of the flexible pipe connected with the longitudinal channel for supply of drugs, and a number of injection holes made in the exterior wall of the radial ring channel or channels, **characterized in that** further comprising an indicator ring placed on the exterior of the distal part of the flexible pipe on a site of location of the injection holes or in the immediate vicinity to the site along a diameter of the flexible pipe; where a color of the indicator ring differs from a color of the exterior, at least, of the distal part of the flexible pipe; and the indicator ring is located so that it can be visible from the side of the laryngopharynx in working position of the endotracheal tube, being in this case at the boundary of the larynx upper part.

2. The tube of claim 1, wherein one end of the longitudinal channel for supply of drugs is connected with a means for supply of drugs, and the second end of the longitudinal channel is connected with the radial ring channel or channels.

3. The tube of claim 2, wherein the longitudinal air channel and the longitudinal channel for supply of drugs are located diametrically opposite one to another within the wall of the flexible pipe.

4. The tube of claim 1, wherein it contains two or more radial ring channels, and a total hydraulic resistance of one radial ring channel together with the injection holes made in it should be equal to a total hydraulic resistance of another radial ring channel together with the injections holes made in it, or every of other radial ring channels together with the injection holes made in it.

5. The tube of claim 1, wherein the exterior wall of the radial ring channel for supply of drugs is made as a protruding ring collar of the flexible pipe.

6. The tube of claim 5, wherein the indicator ring is made on the ring collar.

7. The tube of any of claims 1-5, wherein the injection holes are directed perpendicularly to the axes of the distal part of the flexible pipe.

8. The tube of any of claims 1-5, wherein the injection holes are directed at an acute angle to the axes of the distal part of the flexible pipe towards the inflatable cuff.

9. The tube of any of claims 1-5, wherein there are three or more injection holes made in the exterior wall of the radial ring channel.

10. The tube of claims 9, wherein the injection holes are located on the diameter of the radial ring channel with an equal step.
